# EUROPÄISCHE PATENTSCHRIFT

(11) **EP 2 089 357 B1**
(45) Veröffentlichungstag und Bekanntmachung des Hinweises auf die Patenterteilung: **21.09.2016**
(21) Anmeldenummer: 07847586.0
(22) Anmeldetag: 30.11.2007
(51) Int. Cl.: C07C 263/10, C07C 265/14

(54) **VERFAHREN ZUR HERSTELLUNG VON ISOCYANATEN**
PROCESS FOR PREPARING ISOCYANATES
PROCÉDÉ DE FABRICATION D'ISOCYANATES

(30) Priorität: 11.12.2006 EP 06125811
(43) Veröffentlichungstag der Anmeldung: 19.08.2009
(73) Patentinhaber: BASF SE, 67056 Ludwigshafen am Rhein (DE)
(72) Erfinder: KNÖSCHE, Carsten, 67150 Niederkirchen (DE); MATTKE, Torsten, 67251 Freinsheim (DE)
(74) Vertreter: BASF IP Association
(86) Internationale Anmeldenummer: PCT/EP2007/063070
(87) Internationale Veröffentlichungsnummer: WO 2008/071564

(56) Entgegenhaltungen:
- EP-A- 0 928 785
- WO-A-2005/115974

## Beschreibung

Die vorliegende Erfindung betrifft ein Verfahren zur Herstellung von Isocyanaten in bestimmten Apparaten.

Die Herstellung von Isocyanaten durch Phosgenierung der korrespondierenden Amine ist bereits seit langem bekannt. Typischerweise unterscheidet man Phosgenierungen in der Gas- und in der Flüssigphase.

Diesen Verfahren ist gemein, dass sich im Laufe der Reaktion Feststoffe bilden. Diese können zu Ablagerungen bis hin zu Verstopfungen führen. Unter anderem bilden sich bei Phosgenierung in der Flüssigphase aus dem in der Reaktion freiwerdenden Chlorwasserstoff und dem eingesetzten Amin schwerlösliche Hydrochloride. Des Weiteren können in diesen Verfahren die in der Reaktion gebildeten Zwischenprodukte wie Carbamoylchloride unter den Reaktionsbedingungen Feststoffe sein.

Bei allen Phosgenierungen können durch Kontakt von Zwischenprodukten als auch den Isocyanaten mit dem eingesetzten Amin feste Harnstoffe gebildet werden. Auch Folgeprodukte des Isocyanates wie Diimide und Isocyanurate sind häufig unter den Reaktionsbedingungen fest.

Um derartige Ausfällungen zu beseitigen schlägt GB 1165831 beispielsweise für die Reaktion von aromatischen oder aliphatischen Aminen in der Gasphase eine Reaktion in einem Reaktor mit Rührvorrichtung vor.

Dies hat den Nachteil, daß mechanische Rührvorrichtungen, insbesondere unter den Bedingungen der Gasphasenphosgenierung, besonders störanfällig sind.

EP 289840 B1 beschreibt die Durchführung einer Gasphasenphosgenierung in einem Reaktionsraum ohne bewegte Teile. Die Abscheidung verstopfender Teilchen wird hier durch eine turbulente Strömung verhindert.

EP 928785 B1 vermindert die Feststoffbildung durch den Einsatz aufwendiger Mikrostrukturmischer.

Aufgabe der vorliegenden Erfindung war es, ein Verfahren zur Verfügung zu stellen, mit dem die Bildung von Ablagerungen in einem Apparat zur Herstellung von Isocyanaten durch Phosgenierung von Aminen vermindert werden.

Die Aufgabe wurde gelöst durch ein Verfahren zur Herstellung von Diisocyanaten durch Umsetzung der korrespondierenden Amine mit Phosgen gegebenenfalls in Gegenwart von Inerten, in dem die Umsetzung zumindest teilweise in einem Apparat stattfindet, dessen Fluidkontakt-aufweisende Oberfläche eine gemittelte Rauhtiefe Rz gemäß DIN EN ISO 4287 (Fassung vom Oktober 1998) von nicht mehr als 10 µm aufweist und dass der arithmetische Mittenrauwert Ra gemäß DIN EN ISO 4287 (Fassung Oktober 1998) nicht mehr als 1,0 µm beträgt.

An Oberflächen mit einer erfindungsgemäß niedrigen Rauhigkeit haften gegebenenfalls gebildete Partikel nicht oder nur geringfüging an, so daß die Neigung zur Bildung von Ablagerungen vermindert bis nicht vorhanden ist.

Bei den Aminen kann es sich um Monoamine, Diamine, Triamine oder höherwertige Amine handeln, bevorzugt um Diamine. Ensprechend ergeben sich die korrespondierenden Monoisocyanate, Diisocyanate, Triisocyanate oder höherwertigen Isocyanate, bevorzugt Diisocyanate.

Die Amine und Isocyanate können aliphatisch, cycloaliphatisch oder aromatisch sein, bevorzugt aliphatisch oder cycloaliphatisch und besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine, an mindestens ein aromatisches Ringsystem gebundene Isocyanatgruppe aufweisen.

(Cyclo)aliphatische Isocyanate stehen im Rahmen dieser Anmeldung kurz für cycloaliphatische und/oder aliphatische Isocyanate.

Beispiele für aromatische Diisocyanate sind bevorzugt solche mit 6 - 20 C-Atomen, beispielsweise monomeres 2,4'- oder 4,4'-Methylen-di(phenylisocyanat (MDI), 2,4-und/oder 2,6-Toluylendiisocyanat (TDI), o-, m- oder p-Xylendiisocyanat (XDI) und 1,5-oder 1,8-Naphtyldiisocyanat (NDI).

Diisocyanate sind bevorzugt (cyclo)aliphatische Diisocyanate, besonders bevorzugt (cyclo)aliphatische Diisocyanate mit 4 bis 20 C-Atomen.

Beispiele für übliche Diisocyanate sind aliphatische Diisocyanate wie 1,4-Tetramethylendiisocyanat, 1,5-Pentamethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylen-diisocyanat, 1,14-Tetradecamethylendiisocyanat, Derivate des Lysindiisocyanates, Tetramethylxylylendiisocyanat (TMXDI), Trimethylhexandiisocyanat oder Tetra-methylhexandiisocyanat, sowie 3 (bzw. 4), 8 (bzw. 9)-Bis(isocyanatomethyl)-tricyclo[5.2.1.0^{2.6}]decan-Isomerengemische, sowie cycloaliphatische Diisocyanate wie 1,4-, 1,3- oder 1,2-Diisocyanatocyclohexan, 4,4'- oder 2,4'-Di(isocyanatocyclo-hexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Bevorzugt sind 1,5-Pentamethylendiisocyanat, 1,6-Diisocyanatohexan, 1-Isocyanato-3,3,5- trimethyl-5-(isocyanato-methyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanat-ocyclohexyl)methan.

Unter den genannten Aminen müssen solche Amine, die in eine Gasphasenphosgenierung eingesetzt werden können, bestimmte Erfordernisse erfüllen:

Für ein Gasphasenphosgenierungsverfahren können solche Amine zur Reaktion zu den korrespondierenden Isocyanaten eingesetzt werden, bei denen das Amin, deren korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 mol%, besonders bevorzugt zu höchtens 1 mol% und ganz besonders bevorzugt zu höchstens 0,5 mol% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,5-Diaminopentan, 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (mol/mol) Gemisch, Diaminobenzol, 2,6-Xylidin, Napthyldiamin (NDA) und 2,4'- oder 4,4'-Methylen(diphenylamin) (MDA) oder Isomerengemische davon. Bevorzugt sind unter diesen die Diamine, besonders bevorzugt ist 2,4- und/oder 2,6-TDA.

Bei der Gasphasenphosgenierung ist es definitionsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff (HCl) bilden, da die resultierenden Aminhydrochloride leicht ausfallen und nur schwer wieder verdampfbar sind.

Das in der erfindungsgemäße Verfahren einsetzbare Phosgen unterliegt prinzipiell keinen Beschränkungen.

Es kann bevorzugt sein, Phosgen mit einem Gehalt von weniger als 50 ppm Brom und Iod einzusetzen, wie es offenbart ist in der WO 01/00569.

Inerte sind Verbindungen, die man als Lösungsmittel in der Flüssigphasenphosgenierung bzw. als intere Beimischung in der Gasphasenphosgenierung einsetzen kann.

Inert bedeutet dabei, daß pro Reaktionsdurchgang weniger als 5 mol% des eingesetzten Lösungsmittels im Verlauf der Reaktion chemisch verändert werden, bevorzugt weniger als 3 mol%, besonders bevorzugt weniger als 2 und ganz besonders bevorzugt weniger als 1 mol%.

Dabei sind als Lösungsmittel Chlorbenzol, o- oder p-Dichlorbenzol, Trichlorbenzol, Chlortoluole, Chlorxylole, Chlorethylbenzol, Chlornaphthaline, Chlordiphenyle, Methylenchlorid, Perchlorethylen, Toluol, Xylole, Hexan, Dekahydronaphthalin, Diethylisophthalat (DEIP) und andere Carbonsäureester, wie sie beispielsweise in der US 5,136,086, Spalte 3, Zeilen 3 bis 18 aufgeführt sind, Tetrahydrofuran (THF), Dimethylformamid (DMF), Benzol und deren Gemische bevorzugt. Auch kann das hergestellte Isocyanat oder ein Stoffstrom des Verfahrens als Lösungsmittel verwendet werden. Besonders bevorzugt ist Chlorbenzol und Dichlorbenzol.

Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Chlortoluol, o-Dichlorbenzol, Toluol, Xylol, Chlornaphthalin, Decahydronaphthalin, Kohlenstoffdioxid oder Kohlenstoffmonoxid, verwendet werden. Bevorzugt wird Stickstoff und/oder Chlorbenzol als Inertmedium verwendet.

Im allgemeinen wird das Inertmedium in einer Menge eingesetzt, so dass das Verhältnis der Gasvolumina von Inertmedium zu Amin bzw. zu Phosgen mehr als 0,0001 bis 30, bevorzugt mehr als 0,01 bis 15, besonders bevorzugt mehr als 0,1 bis 5 beträgt.

Reaktionsbedingungen Gasphasenphosgenierung

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Die Ausgangsamine werden vor der Durchführung des erfindungsgemäßen Verfahrens verdampft und auf 200°C bis 600°C, vorzugsweise 300°C bis 500°C erhitzt und gegebenenfalls verdünnt mit einem Inertgas oder mit den Dämpfen eines inerten Lösungsmittels durch die Mischeinrichtung dem Reaktor zugeführt. Unter Reaktor wird die technische Vorrichtung verstanden, die den Reaktionsraum enthält.

Das bei der Phosgenierung verwendete Phosgen wird vor Durchführung des erfindungsgemäßen Verfahrens gegebenenfalls verdünnt mit einem Inertgas oder mit den Dämpfen eines inerten Lösungsmittels ebenfalls auf eine Temperatur innerhalb des Bereichs von 200°C bis 600°C, vorzugsweise 300°C bis 500°C erhitzt.

Die Umsetzung von Phosgen mit Amin im Reaktionsraum in der Gasphasenphosgenierung erfolgt in der Regel bei Absolutdrücken von mehr als 0,1 bar bis weniger als 20 bar, bevorzugt zwischen 0,5 bar und 15 bar und besonders bevorzugt zwischen 0,7 und 10 bar. Im Fall der Umsetzung von (cyclo)aliphatischen Aminen beträgt der Absolutdruck ganz besonders bevorzugt zwischen 0,7 bar und 5 bar, insbesondere von 0,8 bis 3 bar und speziell 1 bis 2 bar. Die Gasphasenphosgenierung kann aber auch bei moderaten Drücken von 3 bis 20 bar erfolgen, wie in der WO 2004/026813 beschrieben.

Reaktionsbedingungen Flüssigphasenphosgenierung

Im Gegensatz dazu findet die Reaktion von Amin und Phosgen in der Flüssigphasenphosgenierung im allgemeinen zwischen 20 °C und 250 °C, bevorzugt zwischen 40 und 230 °C statt. Der Druck beträgt in der Regel zwischen 1,0 bar und 80 bar abs.

Entscheidend dabei ist, daß die Reaktion von Amin mit Phosgen zu einem überwiegenden Teil in der Flüssigphase stattfindet. Daneben kann durchaus auch eine Gasphase vorliegen, die beispielsweise überwiegend aus Chlorwasserstoff besteht.

Unabhängig von der Reaktionsführung als Flüssigphasen- oder Gasphasenphosgenierung werden die Ausgangsverbindungen zunächst in mindestens einem Mischorgan miteinander vermischt und anschließend in mindestens einem Reaktionsraum zur Reaktion gebracht. Der Reaktionsaustrag aus dem Reaktionsraum wird in der Gasphasenphosgenierung zum Abstoppen der Reaktion anschließend in einem sogenannten "Quench" durch Inkontaktbringen mit einem Lösungsmittel abgekühlt.

Der Austrag aus dem Reaktionsraum bei der Flüssigphasenphosgenierung bzw. der Austrag aus dem Quench bei der Gasphasenphosgenierung wird dann zumeist destillativ aufgereinigt.

Reaktionsführung Flüssigphasenphosgenierung

Die Vermischung der Eduktströme, wobei das Amin auch als Hydrochlorid eingesetzt werden kann, erfolgt häufig in einer geeigneten speziellen Mischeinrichtung, die sich durch geringe Mischzeiten auszeichnet.

Die Mischzeit in solchen Mischeinrichtungen beträgt üblicherweise von 0,0001 s bis 2 s, bevorzugt von 0,0005 bis 1 s, besonders bevorzugt von 0,001 bis 0,5 s, ganz besonders bevorzugt von 0,005 bis 0,2 s und insbesondere von 0,007 bis 0,1 s.

Als Mischzeit ist diejenige Zeit zu verstehen, die von dem Beginn des Mischvorgangs vergeht, bis 97,5 % der Fluidelemente des erhaltenen Gemisches einen Mischungsbruch haben, der bezogen auf den Wert des theoretischen Endwert des Mischungsbruchs des erhaltenen Gemisches beim Erreichen des Zustandes perfekter Mischung weniger als 2,5 % von diesem Endwert des Mischungbruches abweichen. (zum Konzept des Mischungsbruches siehe z.B. J.Warnatz, U.Maas, R.W. Dibble: Verbrennung, Springer Verlag, Berlin Heidelberg New York, 1997, 2. Auflage, S. 134.)

Als Mischeinrichtung bevorzugt wird ein Mischkreis, ein Rührbehälter, eine Mischpumpe oder eine Düsenmischeinrichtung, beispielsweise Koaxialmischdüsen, Y- oder T-Mischer, oder eine Vortex-Impinging-Jet-Mischkonfiguration eingesetzt, bevorzugt ein Mischkreis, ein Rührbehälter, eine Mischpumpe oder eine Düsenmischeinrichtung.

Bei der Verwendung eines Mischkreises oder eines Rührbehälters als Mischeinrichtung ist es wichtig, dass die Aminlösung mit hoher Geschwindigkeit eingedüst wird. Üblicherweise liegen die Geschwindigkeiten zwischen 10 und 100 m/s, bevorzugt zwischen 20 und 80 m/s.

Bevorzugt wird eine Mischdüse und eine Mischpumpe als Mischeinrichtung eingesetzt. Besonders bevorzugt wird als Mischeinrichtung eine Mischdüse verwendet. Hierbei ist es wichtig, dass sowohl der Phosgen- als auch der Amineduktstrom mit hoher Geschwindigkeit in die Mischdüse eingeleitet werden. Die Geschwindigkeiten betragen zwischen 10 und 100 m/s, bevorzugt zwischen 20 und 80 m/s.

Dabei liegt der Druck in der Amin- und Phosgenzuleitung zur Düse erheblich höher als in Ausgang der Mischdüse, üblicherweise jedoch nicht höher als 110 bar abs, bevorzugt nicht höher als 100 bar abs, besonders bevorzugt 10 bis 95 bar abs und ganz besonders bevorzugt 15 bis 90 bar abs.

Der Druck am Ausgang der Mischeinrichtung liegt zwischen 10 und 100 bar, bevorzugt zwischen 15 und 80 bar, besonders bevorzugt zwischen 30 und 70 bar.

Die Temperatur des Austrages aus der Mischeinrichtung beträgt in der Regel zwischen 25 und 240 °C, bevorzugt 30 - 190 und besonders bevorzugt 40 - 160 °C. Der Austrag aus der Mischeinrichtung kann vor Einleiten in den Reaktionsraum mit Hilfe eines Wärmetauschers auf die dort gewünschte Temperatur gebracht werden.

Im Reaktionsraum wird die Reaktion vervollständigt und bereits ein Großteil des gebildeten Carbamoylchlorids zum Isocyanat gespalten.

Der Reaktionsraum kann rückvermischte oder nicht-rückvermischte Reaktoren sowie Reaktivdestillationskolonnen umfassen.

Rückvermischte Reaktoren sind beispielsweise Rührkessel, Rührkesselkaskaden aus 2 bis 4 Rührkesseln, Schlaufenreaktoren oder ungerührte Behälter.

Weitestgehend-rückvermischunsgfreie Reaktoren sind beispielsweise Rohrreaktoren. Dies wird beispielsweise erreicht durch das Verhältnis des Durchmessers des Rohrreaktors zu dessen Länge oder durch Einbauten, wie Lochböden, Schlitzböden oder statische Mischer. Bevorzugt wird die Rückvermischungsfreiheit durch das Verhältnis von Länge zu Durchmessers des Rohrreaktors erreicht. Als Rohrreaktor eignen sich alle Rohre, deren Längen- zu Durchmesserverhältnis größer als 5 ist, bevorzugt größer als 6, besonders bevorzugt größer als 10.

Eine während der Reaktion anfallende Gasphase kann im Gleich- oder Gegenstrom zur flüssigen Phase bewegt werden.

Reaktivdestillationskolonnen sind von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Bevorzugt werden Böden verwendet, besonders bevorzugt Glockenböden.

Üblicherweise weisen derartige Kolonnen 10 - 80 theoretische Trennböden auf.

Die mittlere Gesamtverweilzeit im Reaktionsraum bei der Flüssigphasenphosgenierung liegt in der Regel zwischen 20 min und 18 h, bevorzugt zwischen 30 min und 15 h, besonders bevorzugt zwischen 50 min und 11 h.

Der Umsatz im Reaktionsraum ist nahezu vollständig.

Reaktionsführung Gasphasenphosgenierung

Zur Reaktion werden bei der Gasphasenphosgenierung die Edukte sowie gegebenenfalls Inertes in der Regel über mindestens eine Mischeinrichtung miteinander vermischt.

Bei der Mischeinrichtung kann es sich bevorzugt um statische Mischorgane, beispielsweise um eine Düsenmischeinrichtung, beispielsweise Koaxialmischdüsen, Y- oder T-Mischer, Strahlmischer oder Mischrohre handeln.

Bei einem Strahlmischer wird in einem Mischrohr durch ein konzentrisches Rohr mit kleinem Duchmesser (Düse) mit hoher Geschwindigkeit die eine Komponente (bevorzugt das Amin) in die andere Komponente (hier Phosgen) geführt.

Bei den Reaktoren kann es sich beispielsweise um zylinderförmige Reaktionsräume ohne Einbauten und ohne bewegte Teile handeln.

Eine Ausführungsform einer Mischungs-/Reaktionseinheit ist beschrieben in EP 1275639 A1, dort besonders in den Absätzen [0013] bis [0021] und dem Beispiel zusammen mit Figur 1, was hiermit durch Bezugnahme Bestandteil der vorliegenden Offenbarung sei. Bevorzugt ist jedoch im Gegensatz zur dortigen Offenbarung die Dosierung des Amin durch das Innenrohr und von Phosgen als äußerem Strom.

Eine Ausführungsform einer Mischungs-/Reaktionseinheit ist beschrieben in EP 1275640 A1, dort besonders in den Absätzen [0010] bis [0018] und dem Beispiel zusammen mit Figur 1, was hiermit durch Bezugnahme Bestandteil der vorliegenden Offenbarung sei. Bevorzugt ist jedoch im Gegensatz zur dortigen Offenbarung die Dosierung des Amin durch das Innenrohr und von Phosgen als äußerem Strom.

Eine weitere Ausführungsform einer Mischungs-/Reaktionseinheit ist beschrieben in EP 1319655 A2, dort besonders in den Absätzen [0015] bis [0018] und dem Beispiel zusammen mit Figur 1.

Es kann sinnvoll sein, Strömungsvergleichmäßiger einzubauen, wie beschrieben in EP 1362847 A2, dort besonders in den Absätzen [0008] bis [0026] und dem Beispiel zusammen mit Figur 1.

Denkbar ist auch der Einsatz von mehreren parallel ausgerichteten Düsen, wie beschrieben in EP 1449826 A1, dort besonders in den Absätzen [0011] bis [0027] und Beispiel 2 zusammen mit den Figuren 1 bis 3.

Es kann sinnvoll sein, den Turbulenzgrad zur Vermischung zu erhöhen durch drallerzeugende Einbauten, wie beschrieben in EP 1526129 A1, dort besonders in den Absätzen [0008] bis [0026] und dem Beispiel zusammen mit den Figuren 1 bis 3.

Eine weitere Ausführungsform einer Mischungs-/Reaktionseinheit ist beschrieben in DE 10359627 A1, dort besonders in den Absätzen [0007] bis [0025] und Beispiel 1 zusammen mit der Figur.

Eine bevorzugte Ausführungsform für eine Mischdüse ist eine Schlitzmischdüse, wie sie beschrieben ist in der Europäischen Patentanmeldung mit dem Aktenzeichen 06123631.1 und dem Anmeldedatum 7.11.2006, dort besonders von Seite 3, Zeile 28 bis Seite 15, Zeile 35 und ein Reaktionsraum wie er dort beschrieben ist von Seite 15, Zeile 39 bis Seite 23, Zeile 38 zusammen mit den Figuren.

Eine besonders bevorzugte Ausführungsform für eine Mischdüse ist eine Ringspaltmischdüse, wie sie beschrieben ist in der Internationalen Patentanmeldung WO 2007/028715, dort besonders von Seite 2, Zeile 23 bis Seite 11, Zeile 22 und ein Reaktionsraum wie er dort beschrieben ist von Seite 11, Zeile 26 bis Seite 21, Zeile 15 zusammen mit Figur 2.

Der Quench kann beispielsweise ausgeführt sein wie beschrieben in EP 1403248 A1, dort besonders in den Absätzen [0006] bis [0019] und dem Beispiel zusammen mit den Figuren 1 bis 2.

Der Quench kann beispielsweise ausgeführt sein wie beschrieben in der Europäischen Patentanmeldung mit dem Aktenzeichen 06123629.5 und dem Anmeldedatum 7.11.2006, dort besonders von Seite 3, Zeile 30 bis Seite 11, Zeile 37 zusammen mit Beispiel 1 und den Figuren.

Der Quench kann beispielsweise ausgeführt sein wie beschrieben in der Europäischen Patentanmeldung mit dem Aktenzeichen 06123621.2 und dem Anmeldedatum 7.11.2006, dort besonders von Seite 3, Zeile 26 bis Seite 16, Zeile 36 zusammen mit Beispiel 1 und den Figuren.

Der Quench kann bevorzugt ausgeführt sein wie beschrieben in WO 2005/123665, dort besonders von Seite 3, Zeile 10 bis Seite 8, Zeile 2 und dem Beispiel.

Erfindungsgemäß wesentlich ist, daß solche Oberflächen des Apparates, in dem die Reaktion stattfindet, die Fluidkontakt haben, eine mittlere Rauhtiefe Rz gemäß DIN EN ISO 4287 (Fassung vom Oktober 1998) von nicht mehr als 10 µm, bevorzugt nicht mehr als 8, besonders bevorzugt nicht mehr als 6, ganz besonders bevorzugt nicht mehr als 4, insbesondere nicht mehr als 2 und speziell nicht mehr als 1 µm aufweisen.

An derartigen erfindungsgemäß wenig rauhen Oberflächen beobachtet man eine geringere Ablagerungsbildung als an stärker rauhen Oberflächen.

Besonders sollten solche Oberflächen eine erfindungsgemäß niedrige Rauhigkeit aufweisen, die mit beiden Edukten Phosgen und Amin Kontakt haben und/oder mit einem der Zwischenprodukte der Reaktion, beispielsweise die korrespondierenden Carbamoylchloride, bevorzugt solche Oberflächen, die mit beiden Edukten Phosgen und Amin Kontakt haben.

Es kann weiterhin sinnvoll sein, solche Oberflächen erfindungsgemäß wenig rauh auszugestalten, die mit lediglich einem Edukt, also entweder Amin oder Phosgen, Kontakt haben, beispielsweise in Zuleitungen.

Es kann weiterhin sinnvoll sein, solche Oberflächen erfindungsgemäß wenig rauh auszugestalten, die mit im wesentlichen reinen Produkt, also dem Isocyanat, Kontakt haben.

In einer bevorzugten Ausführungsform ist zumindest die Oberflächen mit Fluidkontakt der Mischeinrichtung erfindungsgemäß wenig rauh ausgestaltet.

In einer besonders bevorzugten Ausführungsform ist zumindest die Oberflächen mit Fluidkontakt des Reaktionsraums erfindungsgemäß wenig rauh ausgestaltet.

Es kann weiterhin sinnvoll sein, zusätzlich den Quench erfindungsgemäß wenig rauh auszustalten.

Mit dem Begriff "Fluid" seien dabei sowohl Flüssig- als auch Gasphase umfaßt.

Zu Unterscheidungszwecken kann als Mischraum der Bereich des Reaktionsraumes bezeichnet werden, in dem die Vermischung der Edukte zu einem Grad von 99% stattfindet. In einer bevorzugten Ausführungsform der vorliegenden Erfindung ist der Umsatz im Mischraum, d.h. der Verbrauch des eingesetzten Amins, weniger als 15%. Dabei wird der Vermischungsgrad als Verhältnis der Differenz des lokal gemittelten Mischungsbruch und des Anfangsmischungsbruchs vor Vermischung zur Differenz des mittleren endgültigen Mischungsbruchs nach Vermischung und des Anfangsmischungsbruchs vor Vermischung angegeben. Zum Konzept des Mischungsbruches siehe z.B. J. Warnatz, U. Maas, R.W. Dibble: Verbrennung, Springer Verlag, Berlin Heidelberg New York, 1997, 2. Auflage, S. 134.

Als Reaktionsraum wird das Volumen beschrieben, in dem mindestens 98% des Umsatzes, d.h. der Verbrauch des eingesetzten Amins, stattfindet, bevorzugt mindestens 99%, besonders bevorzugt 99,5%, ganz besonders bevorzugt 99,7%, insbesondere 99,9% und speziell 99,99%.

Neben den angegebenen gemittelten Rauhtiefen Rz sind weitere Parameter einzuhalten, beispielsweise die arithmetischen Mittenrauhwerte Ra gemäß DIN EN ISO 4287 (Fassung vom Oktober 1998), die nicht mehr als 1,0 µm betragen, und bevorzugt nicht mehr als 0,8 µm.

Die Werte für Rz sowie gegebenenfalls Ra werden bevorzugt mit elektrischen Tastschnittgeräten abgenommen. Dies erfolgt bevorzugt gemäß DIN EN ISO 4288.

Dazu ertastet ein elektrisches Tastschnittgerät mit einer hochempfindlichen Diamant-Tastspitze das Profil längs der genormten Taststrecke. Es wird dabei automatisch eine Bezugslinie gemittelt, die sogenannte mittlere Linie. Grundsätzlich werden mit dem Gerät eine zweidimensionale Erfassung des angenäherten Abbildes der Werkstückoberfläche erfasst. Die hochempfindliche Tastspitze fährt während der Rauheitsmessung über die Oberflächenkontur.

Weitere Messverfahren sind prinzipiell bekannt und können der Fachliteratur (beispielsweise Dubbel "Taschenbuch für den Maschinenbau" , Springer-Verlag) entnommen werden.

Geeignete Materialien für den Kontakt mit dem Reaktionsgemisch sind z.B. Metalle, wie Stahl, insbesondere legierter Stahl, Tantal, Nickel, Nickellegierungen, Silber oder Kupfer, Glas, Keramik, Emaille oder homogene oder heterogene Gemische und Bauteile daraus. Bevorzugt werden Stahlapparate, besonders bevorzugt -reaktoren verwendet.

Dabei ist es bevorzugt, die Apparate zumindest teilweise an den thermisch belasteten Stellen, an denen der Apparat dem Reaktionsgemisch ausgesetzt ist, aus korrosionsbeständigen Werkstoffen zu fertigen. Dazu gehören insbesondere nichtrostende Stähle und Nickel-Knetlegierungen.

Die DIN-EN 10088-1 in der Fassung vom August 1995 definiert nichtrostende Stähle als solche, die mindestens 10,5 % Chrom und höchstens 1,2 % Kohlenstoff enthalten. Bevorzugt werden dann erfindungsgemäß Apparate eingesetzt, die zumindest teilweise aus austenitischen und/oder austenitisch-ferritischen Stählen gefertigt sind.

Austenitische Stähle sind solche mit austenitischem Gittertyp (γ-Phase) bei 20 °C. Bevorzugt weisen sie einen Cr-Gehalt von 16 bis 28 % und einen Ni-Gehalt von 3,5 bis 32 % auf, sowie gegebenenfalls Anteile von S (bis zu 0,35%), P (bis zu 0,045%), Mo (bis zu 7%), Si (bis zu 4,5%), Cu (bis zu 4%), N (bis zu 0,25%) und/oder Mn (bis zu 10,5%), sowie eventuell Ti (bis zu 0,7%) und/oder Nb (bis zu 1 %). Der Kohlenstoffgehalt beträgt in der Regel unter 0,15%. Unter diesen sind die hochlegierten austenitischen 18/8 Chrom-Nickelstähle besonders bevorzugt.

Austenitisch-ferritische Stähle weisen ein Zweiphasengefüge aus Ferrit und Austenit mit einem Ferrit-Anteil von ca. 60% auf. Der Cr-Anteil beträgt meist 19 - 28 %, Ni 3,5 - 8%, Mo bis zu 4,5% und gegebenenfalls Anteile von Mn (bis zu 2%), Cu (bis zu 2,5%), N (bis zu 0,35%), W (bis zu 1 %), S (bis zu 0,015%), Si (bis zu 1 %)und/oder P (bis zu 0,035%). Der Kohlenstoffgehalt beträgt in der Regel unter 0,05%.

Ganz besonders bevorzugte Werkstoffe sind die in der DIN-EN 10088-1 aufgeführten austenitischen und austenitisch-ferritischen Werkstoffe und insbesondere bevorzugt sind die Werkstoffe 1.4539 (Falk-Stahl), 1.4541, 1.4571 und 1.4462, sowie ferner auch Hastelloy A und C und Zirkon. Die genannten Werkstoffe gemäß DIN-EN 10088 entsprechen näherungsweise folgenden Werkstoffen gemäß AISI (American Iron and Steel institute), UNS (Unified Numbering System), SS (Swedish Standard), AFNOR (Association Francaise de Normalisation), BS (British Standard) und JIS (Japanese Industrial Standards):

1.4462 (X 2 CrNiMoN 22 5 3): UNS: S 31803, SS: 2377, AFNOR: Z 5 CNDU 21.08, JIS: SUS 329 J3L

1.4539 (X 1 NiCrMoCuN 25 20 5): UNS: N 08904, SS: 2562, AFNOR: Z 1 NCDU 25.20 1.4541 (X 6 CrNiTi 18 10): AISI: 321, UNS: S 32100, SS: 2337, AFNOR: Z 6 CNT 18.10, BS: 321 S 31, JIS: SUS 321

1.4571 (X 6 CrNiMoTi 17 12 2): AISI: 316 Ti, UNS: S 31635, SS: 2350, AFNOR: Z 6 CNDT 17.12, BS: 320 S 31, JIS: SUS 316 Ti

Unter den angeführten Werkstoffen sind solche mit erhöhten Chrom-, Kupfer-, Molybdän- und/oder Nickelanteilen vorteilhaft.

Neben den o.g. Edelstählen können ebenso Nickel und Nickel-Knetlegierungen (Werkstoffbezeichnungen 2.4xxx gemäß DIN 17744:2002-09) eingesetzt werden. Dabei werden bevorzugt temperatur- und korrosionsbeständige Legierungen mit einem Molybdängehalt von mehr als 6 Gew% bis zu 25 Gew% und einem Chromgehalt von mindestens 6 Gew% bis zu 26 Gew% genutzt. Neben geringen Mengen anderer Metalle können diese Werkstoffe höhere Anteile von Kobalt, Kupfer, Eisen, Mangan, Niob und/oder Tantal sowie Wolfram enthalten.

Des weiteren sind kupferhaltige Nickel-Knetlegierungen mit den Werkstoffbezeichnungen 2.4xxx gemäß DIN 17744:2002-09 einsetzbar. Diese enthalten neben Nickel vor allem Kupfer sowie neben geringen Mengen anderer Metalle können diese Werkstoffe höhere Anteile von Eisen.

Besonders bevorzugt sind folgende Werkstoffe: 2.4602 (NiCr21 Mo14W), 2.4606 (NiCr21 Mo16W), 2.4610 (NiMo16Cr16Ti), 2.4619 (NiCr22Mo7Cu), 2.4819 (Ni-Mo16Cr15W), 2.4856 (NiCr22Mo9Nb), 2.4360 (NiCu30Fe) sowie 2.4361 (LC-NiCu30Fe).

Diese Werkstoffe werden häufig unter den Handelsnamen HASTELLOY®, INCONEL®, MONEL® und anderen angeboten.

Die Apparate mit den erfindungsgemäß niedrigen Rauhigkeiten werden in der Regel durch eine mehrstufige Fertigung mit trennenden Fertigungsverfahren hergestellt. So können sich dem ersten Fertigungsschritt wie Bohren, Fräsen oder Drehen weitere Fertigungsschritte anschließen. Dies können ebenfalls Bohren, Fräsen oder Drehen, jedoch dann mit veränderten Parametern, z.B. einer verminderten Vorschubgeschwindigkeit sein (Feinschlichten). Andere trennende Fertigungsverfahren wie Schleifen, mechanisches und/oder Elektropolieren, Honen oder Läppen können ebenfalls zum Einsatz kommen. Alternativ können rauhe Oberflächen jedoch auch durch auftragende Verfahren wie Beschichten, Platieren, Galvanisieren o.ä. geglättet werden.

Mit Apparaten, die eine erfindungsgemäße geringe Rauhigkeit aufweisen, kann die Bildung von Ablagerungen in der Flüssigphasenphosgenierung und Gasphasenphosgenierung, bevorzugt in der Gasphasenphosgenierung vermindert werden.

Die Erfindung soll anhand der folgenden Beispiele erläutert werden:

### Beispiel 1 (Vergleich)

In einer Versuchsanlage zur Phosgenierung eines Isomerengemisches von Toluylendiamin (Verhältnis 2,4- zu 2,6-Isomer ca. 80:20) zu Toluylendiisocyanat in der Flüssigphase wurde eine Gemisch aus Toluylendiamin und Monochlorbenzol über eine zentrale Kapillare von 0,6 mm geführt. In einem Ringspalt um diese Kapillare wird Phosgen im molaren Überschuss zum Amin geführt. Die Kanäle münden in einem Reaktionskessel in der Form, dass die Mündungen in der flüssigen Reaktionsmischung getaucht sind.

Beim Betrieb der Anlage kam es innerhalb weniger Stunden zu einer Verstopfung der Kapillarmündung mit Feststoffen. Die Mittenrauhigkeitswert Ra für die Düse (gemessen an der Außenseite der Innenkapillare nach DIN EN ISO 4288, 4287, 3274 ) betrug 1,97 µm.

### Beispiel 2 (erfindungsgemäß)

Nach Beschichtung der Kapillare aus Beispiel 1 mit einem Glasüberzug mit einer Mittenrauhigkeit Ra von ca. 0,54 µm konnte ein vierwöchiger stabiler Betrieb ohne Verstopfungen erreicht werden.

## Patentansprüche

1. Verfahren zur Herstellung von Diisocyanaten durch Umsetzung der korrespondierenden Amine mit Phosgen gegebenenfalls in Gegenwart von Inerten, **dadurch gekennzeichnet, daß** die Umsetzung zumindest teilweise in einem Apparat stattfindet, dessen Fluidkontakt-aufweisende Oberfläche eine gemittelte Rauhtiefe Rz gemäß DIN EN ISO 4287 (Fassung vom Oktober 1998) von nicht mehr als 10 µm aufweist und dass der arithmetische Mittenrauwert Ra gemäß DIN EN ISO 4287 (Fassung Oktober 1998) nicht mehr als 1,0 µm beträgt.

2. Verfahren gemäß Anspruch 1, **dadurch gekennzeichnet, daß** das Isocyanat ausgewählt ist aus der Gruppe bestehend aus 1,5-Pentamethylendiisocyanat, 1,6-Diisocyanatohexan, 1-Isocyanato-3,3,5- trimethyl-5-(isocyanatomethyl)cyclohexan, 4,4'-Di(isocyanatocyclohexyl)methan und Toluylendiisocyanat-Isomerengemischen.

3. Verfahren gemäß einem der vorstehenden Ansprüche, **dadurch gekennzeichnet, daß** bei der Umsetzung die Eduktströme und Zwischenprodukte im gasförmigen Zustand miteinander zu den Produkten reagieren und im Verlauf der Reaktion während des Durchgangs durch den Reaktionsraum zu mindestens 95% in der Gasphase bleiben.

4. Verfahren gemäß einem der Ansprüche 1 oder 2, **dadurch gekennzeichnet, daß** die Reaktion von Amin und Phosgen zwischen 20 °C und 250 °C und einem Druck zwischen 1,0 bar und 80 bar abs erfolgt.

5. Verfahren gemäß einem der vorstehenden Ansprüche, **dadurch gekennzeichnet, daß** die gemittelte Rauhtiefe Rz gemäß DIN EN ISO 4287 (Fassung vom Oktober 1998) nicht mehr als 4 µm beträgt.

6. Verfahren gemäß einem der vorstehenden Ansprüche, **dadurch gekennzeichnet, daß** die Oberfläche aus einem Werkstoff besteht ausgewählt ist aus der Gruppe bestehend aus Stahl, legiertem Stahl, Tantal, Nickel, Nickellegierungen, Silber, Kupfer, Glas, Keramik, Emaille und homogene oder heterogene Gemische und Bauteile daraus.

7. Verfahren gemäß einem der vorstehenden Ansprüche, **dadurch gekennzeichnet, daß** es sich bei dem Apparat mit wenig rauher Oberfläche zumindest um den Reaktor handelt

8. Verwendung von Mischeinrichtungen, Reaktoren und/oder Quenchapparaten mit einer Fluidkontakt-aufweisenden Oberfläche, die eine gemittelte Rauhtiefe Rz gemäß DIN EN ISO 4287 (Fassung vom Oktober 1998) von nicht mehr als 10 µm
aufweist und dass der arithmetische Mittenrauwert Ra gemäß DIN EN ISO 4287 (Fassung Oktober 1998) nicht mehr als 1,0 micrometer beträgt, in der Flüssigphasenphosgenierung oder Gasphasenphosgenierung.

## Claims

1. A process for preparing diisocyanates by reaction of the corresponding amines with phosgene, if appropriate in the presence of inerts, wherein the reaction takes place at least partly in an apparatus whose fluid contact surface has an average peak-to-valley height Rz in accordance with DIN EN ISO 4287 (October 1998 version) of not more than 10 µm and wherein the arithmetic mean roughness value Ra in accordance with DIN EN ISO 4287 (October 1998 version) is not more than 1.0 µm.

2. The process according to claim 1, wherein the isocyanate is selected from the group consisting of pentamethylene 1,5-diisocyanate, 1,6-diisocyanatohexane, 1-isocyanato-3,3,5- trimethyl-5-(isocyanatomethyl)cyclohexane, 4,4'-di(isocyanatocyclohexyl)methane and tolylene diisocyanate isomer mixtures.

3. The process according to either of the preceding claims, wherein, in the reaction, the starting material streams and intermediates react with one another to form the products in the gaseous state and remain in the gas phase to an extent of at least 95% during the reaction while passing through the reaction space.

4. The process according to either claim 1 or 2, wherein the reaction of amine and phosgene occurs at from 20°C to 250°C and a pressure of from 1.0 bar to 80 bar abs.

5. The process according to any of the preceding claims, wherein the average peak-to-valley height Rz in accordance with DIN EN ISO 4287 (October 1998 version) is not more than 4 µm.

6. The process according to any of the preceding claims, wherein the surface comprises a material selected from the group consisting of steel, alloy steel, tantalum, nickel, nickel alloys, silver, copper, glass, ceramic, enamels and homogeneous or heterogeneous mixtures and components made thereof.

7. The process according to any of the preceding claims, wherein the apparatus having a surface of low roughness is at least the reactor.

8. The use of mixing devices, reactors and/or quench apparatuses having a fluid contact surface which has an average peak-to-valley height Rz in accordance with DIN EN ISO 4287 (October 1998 version) of not more than 10 µm and wherein the arithmetic mean roughness value Ra in accordance with DIN EN ISO 4287 (October 1998 version) is not more than 1.0 micrometer in liquid-phase phosgenation or gasphase phosgenation.

## Revendications

1. Procédé de fabrication de diisocyanates par mise en réaction des amines correspondantes avec du phosgène, éventuellement en présence de composés inertes, **caractérisé en ce que** la réaction a lieu au moins en partie dans un appareil dont la surface présentant un contact avec le fluide présente une profondeur de rugosité moyenne Rz selon DIN EN ISO 4287 (version d'octobre 1998) inférieure ou égale à 10 µm, et **en ce que** la valeur de rugosité moyenne arithmétique Ra selon DIN EN ISO 4287 (version d'octobre 1998) est inférieure ou égale à 1,0 µm.

2. Procédé selon la revendication 1, **caractérisé en ce que** l'isocyanate est choisi dans le groupe constitué par le diisocyanate de 1,5-pentaméthylène, le 1,6-diisocyanatohexane, le 1-isocyanato-3,3,5-triméthyl-5-(isocyanato-méthyl)cyclohexane, le 4,4'-di(isocyanatocyclohexyl)méthane et les mélanges d'isomères de diisocyanate de toluylène.

3. Procédé selon l'une quelconque des revendications précédentes, **caractérisé en ce que**, lors de la réaction, les courants de réactifs et les produits intermédiaires sont mis en réaction les uns avec les autres à l'état gazeux pour former les produits, et restent en phase gazeuse à hauteur d'au moins 95 % au cours de la réaction pendant la traversée de la chambre de réaction.

4. Procédé selon l'une quelconque des revendications 1 ou 2, **caractérisé en ce que** la réaction de l'amine et du phosgène a lieu entre 20 °C et 250 °C, et à une pression comprise entre 1,0 bar et 80 bar abs.

5. Procédé selon l'une quelconque des revendications précédentes, **caractérisé en ce que** la profondeur de rugosité moyenne Rz selon DIN EN ISO 4287 (version d'octobre 1998) est inférieure ou égale à 4 µm.

6. Procédé selon l'une quelconque des revendications précédentes, **caractérisé en ce que** la surface est constituée d'un matériau choisi dans le groupe constitué par l'acier, l'acier allié, le tantale, le nickel, les alliages de nickel, l'argent, le cuivre, le verre, la céramique, les émaux et les mélanges homogènes et hétérogènes et les composants de ceux-ci.

7. Procédé selon l'une quelconque des revendications précédentes, **caractérisé en ce que** l'appareil à surface moins rugueuse est au moins le réacteur.

8. Utilisation de dispositifs de mélange, de réacteurs et/ou d'appareils de trempe ayant une surface présentant un contact avec le fluide qui présente une profondeur de rugosité moyenne Rz selon DIN EN ISO 4287 (version d'octobre 1998) inférieure ou égale à 10 µm et dont la valeur de rugosité moyenne arithmétique Ra selon DIN EN ISO 4287 (version d'octobre 1998) est inférieure ou égale à 1,0 µm, dans la phosgénation en phase liquide ou la phosgénation en phase gazeuse.
